# EUROPEAN PATENT APPLICATION

(11) **EP 4 765 141 A1**
(43) Date of publication of application: **24.06.2026**
(21) Application number: 24307258.4
(22) Date of filing: 20.12.2024
(51) Int. Cl.: G16H 20/10, G16H 70/40

(54) **METHOD FOR DETERMINING AN OPTIMAL DRUG DOSING REGIMEN**

(71) Applicant: Assistance Publique - Hôpitaux de Paris, 75012 Paris (FR); Université Paris Cité, 75006 Paris (FR)
(72) Inventor: HIRT, Déborah, 75014 PARIS (FR)
(74) Representative: Gevers & Orès

(57) **Abstract**

Method implemented by a computer for determining at least one optimal drug dosing regimen to treat a patient (2) efficiently against at least one drug-sensitive pathogenic agent responsible for infection, said method comprising the following steps:
a. obtaining patient-related data related to said patient (2), such as birth date, gender, weight, creatinemia, and co-treatments;
b. obtaining drug-related data, said drug-related data including parameters selected from :
i. the drug, the route of administration when several routes exist for said drug and the adaptation type as "a posteriori" or "a priori" ;
ii. the drug treatment regimen;

wherein the process comprises a next step of:
c. determine values for pharmacokinetic parameter for efficacy PPE and pharmacokinetic parameter for toxicity PPT from the patient related data, the drug related data and at least one population pharmacokinetic model for "a priori" adaptation and for "a posteriori" adaptation, when PPE and PPT are not obtained directly with the measured concentration(s);
d. obtaining infection related data comprising parameters selected from the documentation of infection, the infection localization, the type of infection, the pathogen if identified, the susceptibility of the pathogen to the antifungigram/antibiogram, and the MIC if determined;
wherein the process further comprises next steps of:
e. Determination of an efficacy target for the drug from at least the infection related data;
f. Determination of a target at risk of toxicity; and,
g. Determination of at least one optimal dosing regimen of the drug by comparison of pharmacokinetic parameter of efficacy PPE with the efficacy target and the pharmacokinetic parameter of toxicity PPT with the risk of toxicity target.

## Description

### 1. Field of the invention

The present invention concerns a method for determining drug dosing regimens, in particular in case the drug is an anti-infective drug.

The invention is intended to assist a physician or a doctor in interpreting measured concentrations of a drug and in deciding on most appropriate doses of the drug to administer to a patient to be treated, taking into account the treated infection.

The invention is particularly well-suited to provide optimal drug dosing regimens that can be adapted individually to each patient, taking into account the characteristics of the patient, the characteristics of the drug used, the characteristics of the pathogenic agent (e.g. bacteria or fungi or viruses) found or suspected in the patient's body. The optimal dose(s) can be provided a priori (before treatment initiation) or a posteriori, by informing measured concentration(s).

The method according to the invention contributes by helping to optimize the treatment of a patient by determining the most appropriate suggested dosing regimen (e.g. dose, dosing intervals and the infusion duration for intravenous administration) that is adapted to the patient.

The invention may apply to any type of drugs or medicines that need to be administered to a patient, such as anti-infective drugs to combat bacterial, fungal or viral infections.

### 2. Prior art

Even though the interpretation of biological tests may be facilitated by existing standards, the interpretation of drug dosing still remains cumbersome, as it generally depends on the time elapsed between the time at which the drug was injected and the time at which its concentration was measured, as well as on the actual relationship between the drug concentration and its actual effects on the patient for the active molecule(s) of the drug, as well as the pathogen for infections to treat.

The physician may interpret dosing results if the drug concentration is measured one or several days after starting the treatment and at a fixed time with regard to the time at which the drug was injected into the patient and if the efficiency target of the treating molecule is defined for the same fixed time after the administration (peak concentration or residual concentration i.e. before the next drug injection).

In those specific cases, the interpretation performed by the physician consists in manually evaluating the current dosage and eventually propose an adaptation of the dose by the rule of three based if the pharmacokinetic is linear but does not allow determining an optimal dosing scheme (dose, interval and perfusion delay for intravenous administration) for the patient in order to optimize his/her treatment.

This represents a loss of opportunity for the patient who can seldom benefit from a drug dosing adaptation and even if such an adaptation is performed, it is not optimal for the reasons as stated above. Under these conditions, it takes time for the patient to obtain efficient drug concentrations, which increases the risk for the patient to develop either resistance to anti-infectious treatments or toxicity issues.

In order to overcome at least some of the above limitations and drawbacks, mathematical tools exploiting Bayesian models have already been proposed to estimate, from the drug concentrations measured in the patient's body, the drug concentration time-course in the patient's body regardless of the drug treatment and to simulate an optimal dosing regimen knowing the target drug concentration to be reached.

For instance, such solutions are implemented in existing software, such as *DoseMe, BestDose* or *Ezequiel.* However, these software are not specific of infectious diseases.

WO 2020/245458 discloses a method and a system for providing a dosing regimen to treat a patient efficiently against drug-sensitive pathogenic agents based on patient-related data and drug concentration measured at a random time in the patient's body, the method estimating the drug concentration time-course based on a Bayesian model. This method does not integrate all cases of infections encountered in clinical practice.

Therefore, there is a need to evaluate the dose administered to the patient in terms of efficacy and toxicity and to determine quickly, automatically and with enhanced accuracy, suggestions of optimal effective and non-toxic dosing regimens of a drug that a physician may choose to administer to a patient in order to provide an anti-infective treatment, the infection being either documented or not that is well adapted to the patient thereby overcoming the above-mentioned drawbacks.

### 3. Disclosure of the invention

The invention has been devised to address one or more of the foregoing concerns.

According to a first aspect of the invention there is provided a method implemented by a computer for determining at least one optimal drug dosing regimen to treat a patient efficiently against at least one drug-sensitive pathogenic agent responsible for infection, said method comprising the following steps:
a. obtaining patient-related data related to said patient (2), such as birth date, gender, weight, creatinemia, and co-treatments;
b. obtaining drug-related data, said drug-related data including parameters selected from :
   i. the drug, the route of administration when several routes exist for said drug and the adaptation type as "a posteriori" or "a priori" ;
   ii. the drug treatment regimen;

wherein the process comprises a next step of:
   c. determine values for pharmacokinetic parameter for efficacy PPE and pharmacokinetic parameter for toxicity PPT from the patient related data, the drug related data and at least one population pharmacokinetic model for "a priori" adaptation and for "a posteriori" adaptation, when PPE and PPT are not obtained directly with the measured concentration(s);
   d. obtaining infection related data comprising parameters selected from the documentation of infection, the infection localization, the type of infection, the pathogen if identified, the susceptibility of the pathogen to the antifungigram/antibiogram, and the MIC if determined;
wherein the process further comprises next steps of:
   e. Determination of an efficacy target for the drug from at least the infection related data;
   f. Determination of a target at risk of toxicity; and,
   g. Determination of at least one optimal dosing regimen of the drug by comparison of pharmacokinetic parameter of efficacy PPE with the efficacy target and the pharmacokinetic parameter of toxicity PPT with the risk of toxicity target.

According to another feature of the present invention, for "a posteriori" adaptation, drug-related data includes dates and times of drug administrations and sampling or the calculated or imposed interval between the administration and the sampling and measured drug concentration(s).

According to another feature of the present invention, the selection of the pathogen agent(s) is made according to:
- all pathogens considered to be drug sensitive with available susceptibility to the drug if the location is unknown and the infection is not documented;
- the main pathogens responsible for the infection if the location and the type of the infection are known but not documented, or
- the identified pathogen if the infection is documented.

According to another feature of the present invention, the method comprises a subsequent step of determining the drug susceptibility SUS of the pathogen agent(s) from the selection step.

According to another feature of the present invention, the susceptibility SUS determined is one of:
- MIC if determined; otherwise,
- Critical concentration "S≤" if the pathogen is sensitive to the drug on the antifungigraml/antibiogram; or,
- Critical concentration "R>" if the pathogen is sensitive to high dose; otherwise
- ECOFF if critical concentrations "S≤" and/or "R>" are unknown.

According to another feature of the present invention, the method comprises a step of determining the target of the drug among a group with at least one plasma target, a target at the level of the localization of the infection.

According to another feature of the present invention, the target is associated with the percentage of protein binding %lia of the drug if target is based on free fraction of the drug and/or the percentage of diffusion of the drug Diff to the infected site.

According to another feature of the present invention, the method comprises a step of determining a drug efficacy coefficient Ceff from published literature concentration/efficacy relationships or based on a selected target.

According to another feature of the present invention, the plasma efficacy target is determined based on the selected pathogen(s), associated susceptibility, determined target of the site and determined drug efficacy coefficient.

According to another feature of the present invention, for a plasma target, the plasma efficacy target is equal to SUS x Ceff, if the plasma target depends on a drug total form.

According to another feature of the present invention, the plasma efficacy target is equal to SUS x Ceff/ (1-%lia), if the plasma target depends on a drug free form.

According to another feature of the present invention, for an infected site target, the plasma efficacy target is equal to SUS x Ceff / Diff.

According to another feature of the present invention, the efficacy target is weighted by a percentage of protein binding of the drug at the localization of infection.

According to another feature of the present invention, an efficacy target for controlling n% of the pathogen-susceptible strains is determined, if a MIC distribution for the pathogen/anti-infective pair is available or is calculated.

According to another feature of the present invention, the toxicity target is defined from a demonstrated concentration relationship toxicity, if known, otherwise the toxicity target is replaced by a high concentration by comparison to the literature reported concentrations.

According to another feature of the present invention, the drug dosing regimen, in the case of "a posteriori" adaptation, is the administered regimen to the patient, and, in the case of "a priori" adaptation, is the intended regimen to be administered or the regimen extracted from the summary of product characteristics adapted to the patient's characteristics or the regimen recommended by known expert committees.

According to another feature of the present invention, the method comprise a further step of displaying :
a. glomerular filtration rate as formulated in the population pharmacokinetics publication and/or time to steady state, from patient related data and/or
b. a fit graph showing the adequacy of the measured concentration in the patient and the expected concentrations obtained by simulation of the population pharmacokinetic model applied to patient related data and drug related data, in the case of "a posteriori" adaptation and/or
c. a time-dependent concentration graph in the case of an "a posteriori" adaptation produced with the dose already administered followed by the simulated doses, or in the case of "a priori adaptation" produced with the dosing regimen according to claim 16 or produced with simulated doses and/or,
d. a table listing different possible administration schemes, their corresponding graphic being displayed when clicking on the line of the table corresponding to a specific scheme.

According to another feature of the present invention, the method comprises an additional step of simulating, for the drug, different dosing scheme, ranging from a minimum daily dose to a maximum recommended dose, by increasing or decreasing doses from an administration scheme to another, by varying dose, interval of use and for intravenous administration duration of infusion.

Another object of the present invention is a system comprising an application server and a client terminal, wherein the said client terminal or the application server is adapted to implement partly or fully the method as described above.

According to a feature of the present invention, the system further comprises a patient database from which the client terminal and/or the application server is adapted to retrieve patient-related data.

According to another feature of the present invention, the system further comprises a third-party database from which the client terminal and/or the application server is adapted to retrieve information on pathogenic agents and/or pharmacokinetic/pharmacodynamic information and/or information on drug interactions.

At least parts of the method according to the invention as described above may be implemented by a computer or the like. Accordingly, the present invention may take the form of an entirely hardware embodiment, an entirely software embodiment (including firmware, resident software, micro-code, *etc*) or an embodiment combining both software and hardware aspects that may all generally be referred to herein as a *"circuit", "module"* or *"system".*

Furthermore, the present invention may take the form of a computer programme product embodied in any tangible medium of expression having computer usable programme code embodied in the medium.

Since the present invention can be implemented in software, the present invention can be embodied as computer readable code for provision to a programmable apparatus on any suitable carrier medium.

Thus, another object of the present invention is a computer programme comprising instructions adapted to implement any step of the method as described above when the programme is executed on a computer.

A tangible carrier medium may comprise a storage medium, such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g. a microwave or radiofrequency signal.

Thus, another object of the present invention is a medium for storing information, removable or not, readable by a computer or a microprocessor and comprising code instructions of a computer programme adapted for implementing any step of the method as described above, when the programme is executed by a computer.

### 4. Brief description of drawings

The invention will now be described according to a particular embodiment of the present invention, by way of example only and with reference to the following drawings, in which:
- **Figure 1** illustrates schematically an example of a system wherein the invention is implemented;
- **Figure 2** is schematic block diagram of a computing device for implementing the method according to the invention;
- **Figure 3** illustrates schematically with a block diagram a main part of the method according to the invention;
- **Figure 4** illustrates an example of dose algorithm for cefepime in adult for an a posteriori adaptation, according to the invention;
- **Figure 5** illustrates how the results are displayed (as a comment, a graphic and a table).

### 5. Detailed description

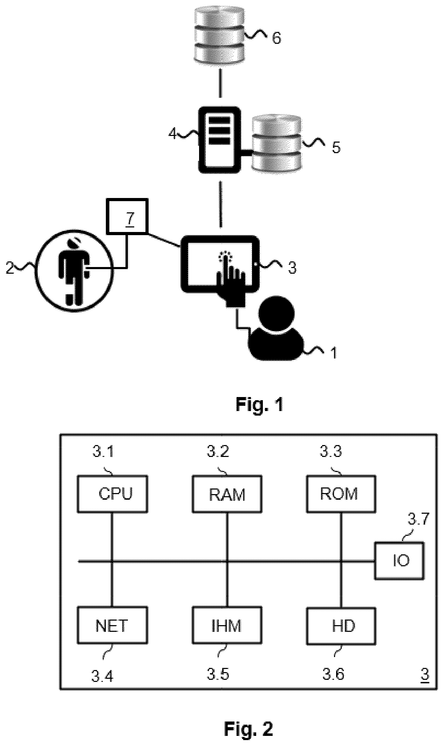

**Figure 1** illustrates schematically a system wherein the present invention may be implemented. For instance, the invention may be implemented in the context of a hospital where a physician or doctor 1 needs to decide on a dosing regimen of drug or medicine to be administered to a patient 2 infected by a pathogenic agent, such as a bacteria or fungi or viruses.

This system comprises a terminal 3 used by the doctor 1, an application server 4, a patient database 5 and third-party databases 6. The patient database 5 comprises information relating to patients and his infection who are being taken care of at the hospital.

The third-party databases 6 may comprise reference information relating to drugs (e.g. antibiotics) and pathogenic agents (e.g. bacteria). For instance, the third-party databases 6 may comprise one or more databases storing information on pharmacokinetics of the drug, on published population pharmacokinetics model, on concentrations - effects relationships, on main pathogens responsible of a type of infection, on bacterial susceptibility, such as the one managed by the *European Committee on Antimicrobial Susceptibility Testing* (EUCAST).

The application server 4 is adapted to retrieve data from both databases 5, 6 via communication networks, such as the Internet (not illustrated). The application server 4 is also adapted to communicate with the terminal 3 via communication networks, such as the Internet and/or an intranet (not illustrated).

The system may further comprise a drug dosing device 7 adapted to measure the drug concentration in the patient's body and/or identified the pathogen, his susceptibility at the antibiogram/antifungigram and/or its minimal inhibitory concentration. The drug dosing device 7 may comprise a wireless communication interface, so as to transmit and/or receive data to/from the terminal 3 via a local area network (not illustrated).

**Figure 2** is a schematic block-diagram of the terminal 3 used for implementing one or more embodiments of the present invention. The terminal 3 may be a laptop, tablet, smartphone or the like adapted to store and execute a programme, software or application, wherein at least part of the method of the present invention may be implemented. For example, the terminal 3 comprises a communication bus connected to:
- a central processing unit (CPU) 3.1, such as a microprocessor;
- a random access memory (RAM) 3.2 adapted to store the executable code for implementing the method of the present invention, as well as registries adapted to save data, variables and parameters necessary for implementing the method according to one or more embodiments of the invention;
- a read only memory (ROM) 3.3 for storing computer programs, software or applications in order to implement the embodiments of the present invention;
- a network interface 3.4 connected to a communication network (intranet, extranet) through which medical data relating to patients, drugs, and/or bacteria may be received and/or transmitted. The network interface 3.4 may be a unique network interface or may comprise a set of different network interfaces (e.g. wired or wireless). Data packets are sent through the network interface or received by the network interface under the control of the application, software or program executed by the processor 3.4;
- a user interface 3.5 for receiving user inputs and/or displaying relevant information to the user (*e.g.* displaying one or more drug doses information to the physician 1);
- an optional storage medium 3.6, such as a hard-drive or memory card (HD);
- an input/output 3.7 for receiving/sending data from/to external peripherals such as a hard-drive, removable storage media or the like.

The executable code may be stored in the read only memory 3.3, in the storage medium 3.6 or on a removable digital medium, such as a memory card.

Alternately, the executable code of the program, software or application may be received in particular from the application server 4, by means of a communication network, through the network interface 3.4, so as to be stored in one of the storage means of the terminal 3, such as the storage medium 3.6, before being executed.

The central processing unit 3.1 is adapted to command and manage the execution of instructions or code portions of the software, application or program according to one of the embodiments of the invention, such instructions being stored in one of the above-mentioned storage means.

After start-up, the CPU 3.1 is able to execute instructions stored in the main RAM 3.2, with regard to the software, application or program, after these instructions have been loaded into the ROM for instance.

Such a software, application or program, when it is executed by the processor 3.1, implements at least some of the steps of the diagram shown in **figure 2****.**

In the preferred embodiment described herein, the terminal 3 is a programmable device that uses a software application configured to implement the method according to the present invention. As an alternative, the present invention may be implemented in hardware, for instance, in the form of a specific integrated circuit or *Application Specific Integrated Circuit* (ASIC).

According to the present embodiment described in reference to **figures 1 and 2** above, the program configured to implement the method of the present invention is hosted and run by the terminal 3.

In alternative embodiments, this program may be hosted and run by the application server 4 or any remote server that has access to the patient database 5 and/or the third-party databases 6. In that case, the terminal 3 may be reduced to a lightweight client that is adapted to connect to the application server or the remote server via a communication network such as the Internet.

The invention will now be described in the context of treating a patient against bacteria. However, the invention is not limited to this particular framework but can also apply to any other pathogenic agents such as viruses or fungi.

For treating a patient against bacteria, the drug or medicine considered to treat the patient is an antibiotic. It is known that antibiotics may be classified in three main categories or classes depending on whether their effect is time-dependent, concentration-dependent or concentration-and-time-dependent.

Time-dependent antibiotics (*e.g.* beta-lactams) are characterized in that their activity on the bacteria is correlated with the duration during which the drug concentration in the patient's body exceeds a given threshold known as the *Minimum Inhibitory Concentration* (MIC).

Concentration-dependent antibiotics (e.g. aminosides) are characterized in that their activity on the bacteria increases with drug concentration. Thus, a maximum or peak drug concentration must be reached in order to obtain an optimal effect on the bacteria.

Concentration-and-time-dependent antibiotics (*e.g.* fluoroquinolones) are characterized in that their activity increases with the area under the drug concentration curve as a function of time. The area under the curve (AUC) is known as the exposure.

By default, the term *concentration* designates the total concentration of a drug in a patient's blood, which is the sum of the concentration of drug molecules in free form (*i.e.* unbound) and the concentration of drug molecules bound to the proteins. This total concentration is either measured in the patient's body by sensors or estimated by means of mathematical tools.

Patient-related data may be inputted by the physician by means of the application through a human-machine interface displayed on the terminal 3. Alternatively, the patient-related data may be retrieved by the application from the patient database 5 via the application server 4 if data for a patient are already stored in this database.

When launching the application corresponding to the method 10 according to the invention, the physician is prompted to input, for instance through the human-machine interface by using the input/output interface 3.7, the patient's unique identifier to access his/her profile. Based on the information already available, the application may prompt the physician to add patient-related data comprised within said profile. The application may also use previous data recorded.

The method 10 gathers patient-related data that may include at least one parameter selected from the group consisting of biological characteristics such as date of birth, gender, weight, creatininemia, category of patient (critically ill, cystic fibrosis, hematological malignancies...) and any covariable having an influence on the drug used to treat the patient. Covariable may include any treatment that may be used in combination with the administration of said drug (*i.e.* co-treatment) and that may have an impact on the pharmacokinetics of said drug, for instance by interacting with said drug.

Further, the method 10 gathers drug-related data that may include the following parameters selected from:
- the drug, the route of administration when several exists for the drug and the adaptation type as "a posteriori" or "a priori" ;
- the drug treatment regimen;
- dates and times of drug administrations and sampling or the interval calculated or imposed between the administration in case of "a posteriori" adaptation. The delay is imposed to a value lower that the time to reach steady state for continuous infusion and,
- measured drug concentration(s) in case of an "a posteriori" adaptation,
- at least one population pharmacokinetic model for "a priori" adaptation and for "a posteriori" adaptation when PPE and PPT are not obtained directly with the measured concentration(s);.

It is to be understood by "a priori" adaptation, the situation where the physician starts the treatment administration to the patient or when the drug concentration in the patient's blood is not available. For the "a priori" adaptation of the drug regimen, the method 10 according to the invention may give the choice to the physician of the starting drug regimen. In a custom mode, the physician enters the dosing scheme he wants to administer to the patient (if charge dose, he enters this dose and the infusion duration dose for intravenous and enters the maintenance dose as: the dose and interval and infusion duration for intravenous). In SPC mode, the method 10 according to the invention uses the lowest dose of the mild or of the severe case indicated in the Summary of Product Characteristics (SPC) available through external databases that the method 10 according to the invention can access. In addition, this dose depends on a glomerular filtration rate calculated. In a third mode, the method 10 according to the invention uses a dose according to Dose expert committee (as COMAI) that proposes a standard or high dosage and discontinuous or continuous administration as appropriate. These doses may not appropriate for renal function.

Further, it is to be understood by "a posteriori" adaptation, the situation in the due course of the treatment regimen, the dosing scheme is the administered regimen to the patient. The "a posteriori" adaptation occurs when at least a concentration is available. According the embodiment of the method 10 according to the invention, the physician is asked if the patient receive a continuous infusion for at least the mean time to arrive to steady state (calculated from the model and patient related data).

If yes, the method 10 according to the invention requires a daily dose and measured concentration(s).

If no, the method 10 according to the invention asks to the physician to enter the date and time of start of treatment and the date and time of last administration. These entries allow to know if the patient's concentrations are at the steady state or not:
o If yes, the method 10 according to the invention requires dose, number of doses per day and infusion duration if intravenous administration.
∘ If no, the method 10 according to the invention asks the physician to enter all doses received (dose, infusion duration if intravenous administration) and the scheduled next time of administration.

The method 10 according to the invention allows adjustments to be made even if the steady state has not been reached.

Before performing an adaptation, the adequacy between patient and the population pharmacokinetic model (PKPOP model) used is checked :
a. Patient characteristics are compared to the characteristics of the patients who were included in the study which allowed to perform the POPPK model for a posteriori and a priori adaptation
b. A graphic allowing the comparison between measured concentration(s) in the patient and simulated concentrations from the POPPK model with same dosing regimen and same patient characteristics for a posteriori adaptation.

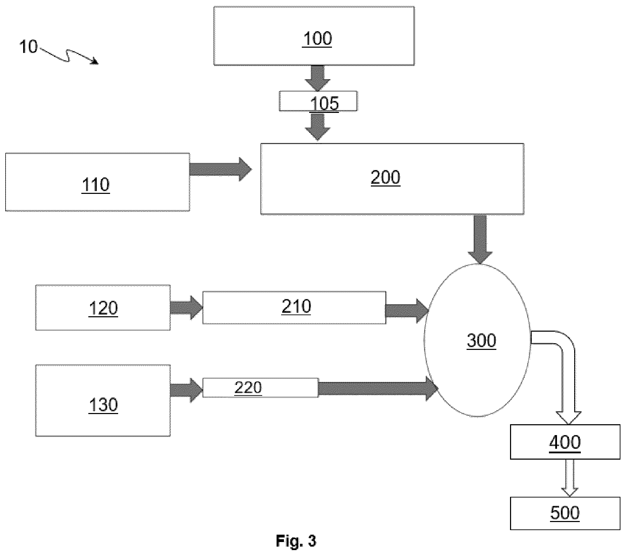

As shown in **Figure 3****,** on a step 100, the method 10 according to the invention obtain further parameters including the documentation of the infection, the infection localization(s) in the patient's body. Additionally, these parameters include the type of infection if the infection is not documented and the identification of the pathogenic agent (e.g. bacteria, fungi, viruses) if identified.

Concerning the documentation of the infection, if the infection is not documented, depending on the drug, the method 10 according to the invention suggests possible localizations of the infection (bronchopulmonary, osteoarticular, urinary...). Depending on this choice, possible types of infection appear. When the physician chooses the type of infection, the method 10 according to the invention searches for main species or genus of bacteria found in these infections (up to three or more). If this localization is not filled, a high molecule dependent target will be used, taking into account pathogens considered to be drug sensitive with available susceptibility data to the drug.

If the infection is documented, the method 10 according to the invention asks for the localization of the infection, pathogen, susceptibility to the antibiogram (sensitive, high dose sensitive) and MIC (minimum inhibitory concentration) if measured. The physician can enter up to three or more different infections or one infection with up to three identified bacteria.

Then, in a step 105, the method 10 according to the invention conducts a selection of the pathogen agent(s) according to:
- all pathogens considered to be drug sensitive with available susceptibility if the location is unknown and the infection is not documented;
- the mains pathogens responsible for the infection if the location and the type of the infection are known but not documented, or
- the identified pathogen if the infection is documented.

On a step 110, the method 10 according to the invention obtain the susceptibility of the identified pathogen to the antifungigram/antibiogram, and the MIC of the identified pathogenic agent if determined. These parameters are completed by data from external databases such as EUCAST recommendations and MIC distributions.

On a subsequent step 200, the method 10 according to the invention determines a drug susceptibility SUS of the pathogen agent(s) from the selection step 105 at least. The data form the step 110 may be also considered. The drug susceptibility SUS determined is one of:
- MIC if determined; otherwise,
- Critical concentration "S≤" if the pathogen is susceptible to antifungigram/antibiogram; or,
- Critical concentration "R>" if the pathogen is sensitive to high dose; otherwise
- ECOFF if critical concentrations "S≤" and/or "R>" are unknown.

If the drug susceptibility SUS is equal to the *Epidemiological Cutt-off Value* (ECOFF), this corresponds to the highest minimal inhibiting concentration for any wild-type bacteria. Such data may be obtained from the data retrieved in existing databases for instance managed by EUCAST. ECOFF values are determined from existing MIC distribution histograms as available on the EUCAST online databases.

Further, a drug susceptibility target for controlling n% of the pathogen-susceptible strains is determined, if a MIC distribution for the pathogen/anti-infective pair is available, susceptible strains being those for which the MIC is lower or equal to the Critical concentration "S≤".

On a step 120, the method 10 according to the invention determines a target of the drug among a group consisting of at least one plasma target, a target at the level of the localization of the infection, the target being associated, in a subsequent step 210, with a percentage of protein binding %lia of the drug and/or a percentage of diffusion of the drug Diff to the infected site.

On a step 220, the method 10 according to the invention determines a drug efficacy coefficient Ceff based on data gathered, in a step 130. Further, the method 10 according to the invention can take into account published concentrations/efficacy relationships in the literature available through databases or based on the target desired by the physician; for example, for beta-lactams, the physician will be able to determine two parameters, j and n, so that the target sought is j% of the time at n x MIC. Indeed, higher targets could be used for more severe patients.

Then, on a step 300, the method 10 according to the invention determines an efficacy target such as an efficacy plasma target for the drug from at least the infection related data from the parameters determined in previous disclosed steps 200,210,220 of the method 10 according to the invention. In particular, the efficacy target is determined based on the selected pathogen(s), associated susceptibility, determined target (plasma, infection site) and determined drug efficacy coefficient.

For a plasma target, if the plasma target depends on a drug total form, the plasma efficacy target is equal to SUS x Ceff.

For a plasma target, if the plasma target depends on a drug free fraction, the plasma efficacy target is equal to SUS x Ceff/ (1-%lia).

For an infected site target, the plasma efficacy target is equal to SUS x Ceff / Diff. Additionally, the efficacy target may be weighted by a percentage of protein binding of the drug at the localization of infection.

It is to be noted that, if multiple pathogens have been identified or suspected, a target of total plasma efficacy will be determined by the method 10 according to the invention to control all susceptible strains of the targeted pathogen(s). Other targets to control at least n% of strains of all identified pathogens will also be proposed when MIC distributions are available.

If neither a critical concentration nor an ECOFF is available, the drug is considered not to cover this pathogen.

The method 10 according to the invention also determines a toxicity target Ctox as follow: if there is a demonstrated concentration versus toxicity relationship, then it is called toxicity, if not, a high concentration is determined by comparison with the concentrations reported in the literature available through external databases.

During step 300, if the patient could be described by the model, the method 10 according to the invention also determines a pharmacokinetic parameter of efficacy PPE and a pharmacokinetic parameter of toxicity PPT from the patient related data, the drug related data and at least one population pharmacokinetic model.

According to the present embodiment, the method 10 according to the invention is configured to define the efficiency pharmacokinetic parameter PPE according to the category of antibiotics used to treat the patient and is further configured to calculate the PPE value based on the results of Bayesian estimation: the latter estimation is made for time-and-concentration dependant anti-infective drug and if the blood tube was not sampled at the good delay (described below) for time dependant and for concentration dependant anti-infective drug.

For time-dependent drugs, the method 10 according to the invention is configured to define the efficiency pharmacokinetic parameter as the total residual drug concentration Cᵣ in the patient's body, just before next administration of the drug to the patient. On another hand, the method 10 according to the invention may define the efficiency pharmacokinetic parameter as a concentration after k% of the time interval between 2 doses (Ck%).

For concentration-dependent drugs, the method 10 according to the invention is configured to define the efficiency pharmacokinetic parameter PPE as the maximum or peak concentration Cₘₐₓ.

For time-and-concentration-dependent drugs, the method 10 according to the invention is configured to define the efficiency pharmacokinetic parameter PPE as the exposure which corresponds to the area under the curve AUC of the drug concentration as a function of time.

The pharmacokinetic parameter of toxicity PPT could be a maximal concentration, a residual concentration (concentration before next dose) or an area under the concentration curve, depending on the concentration - toxicity relationships published in the literature.

These values of PPE or PPT were obtained using Monte Carlo simulation for a priori adaptation, or Bayesian estimation for a posteriori adaptation.

The population pharmacokinetics model describes for each parameter its value, its interindividual variability and the relationship between the covariate and the parameter. Monte Carlo simulations may be used by the method 10 according to the invention, it consists of:
- a draw of n sets of random values of the pharmacokinetic parameters, generated from the statistical distributions (normal, log-normal...), centred on the value of the population parameter applied to the patient (parameter that takes into account the covariate) with a variance corresponding to the interindividual variability of the parameter in the study population.
- each set of values allows to simulate a concentration from the equation of the underlying pharmacokinetic model (for example 1 compartment with 1st order absorption and elimination), adding a residual variability on the concentration.

These types of simulations allow to determine the median Cmax, Cr, Ck% or AUC depending on the case, as well as the prediction interval at 95% of these values

In an embodiment, the method 10 according to the invention carried out a Bayesian adaptation, taking into account both the population curve, covariates, variability but also the concentration or concentrations measured in the patient. Thus, a most likely Cmax, Cr, Ck% or AUC is obtained and a prediction interval is also built, through simulations, if administration is not done continuously. Bayesian adaptation is the minimization of a function that takes into account parameters as the difference between the measured concentration(s) in the patient and the predicted one(s) by the model at the same time weighted by variance of residual variability and the difference between individual pharmacokinetic parameters and population parameters, weighted by the variance of interindividual variability of the parameter.

These methods allow calculating the values of the PPE and PPT parameters (Cmax, Cr, Ck%, AUC).

On a next step 400, the method 10 according to the invention determines with the proposed or given dose in which case is the patient : normodosage, underdosage, overdosage or underdosage with risk of toxicity by comparison of pharmacokinetic parameter of efficacy PPE with the efficacy target and the pharmacokinetic parameter of toxicity PPT with the risk of toxicity target Ctox.

During this step 400, the patient parameters (Cmax, Cr, Ck%, AUC) are then compared by the method 10 according to the invention to plasma targets for efficacy and target for toxicity. For continuous perfusion, only one parameter value will be available, otherwise a 95% prediction interval is determined (note that even for continuous perfusion, a prediction interval may be determined). According to the type of drug:
a. for time dependent drug : PPE = Cr or Ck% compared to efficacy target and PPT = Cr or Cmax compared to toxicity target Ctox;
b. for concentration dependent drug: PPE = Cmax compared to efficacy target, and PPT = Cr compared to toxicity target Ctox; and,
c. for time and concentration dependent drug: PPE = AUC compared to efficacy target, and PPT = Cr or AUC compared to elevated reported concentrations.

The choice of these parameters (Cmax, Cr, Ck%, AUC) to represent pharmacokinetic parameter of efficacy PPE and pharmacokinetic parameter of toxicity PPT may be influenced by the literature data. Similarly, the 5% risk proposed for the prediction interval may be modified.

In the case of continuous infusion with a single available value, the method 10 according to the invention distinguishes four cases:
- Normodosing : PPE ≥ efficacy target & PPT < toxicity target Ctox;
- Underdosing: PPE < efficacy target & PPT < toxicity target Ctox;
- Overdosing: PPE ≥ efficacy target & PPT ≥ toxicity target Ctox;
- Underdosing and toxicity risk : PPE < efficacy target & PPT ≥ toxicity target Ctox;

Otherwise, the method 10 according to the invention considers four cases for the "a priori" adaptation, as for the "a posteriori" adaptation:
- Normodosing: 2.5e percentile (p.) PPE ≥ efficacy target & 97.5 p. PPT < toxicity target Ctox;
- Underdosing with toxicity risk: 2.5e p PPE < efficacy target & 97.5e p PPT ≥ toxicity target Ctox;
- Underdosing: 2.5e p PPE < efficacy target & 97.5e p PPT < toxicity target Ctox;
- Overdosing: 2.5e p. PPE ≥ efficacy target & 97.5e p. PPT ≥ toxicity target Ctox.

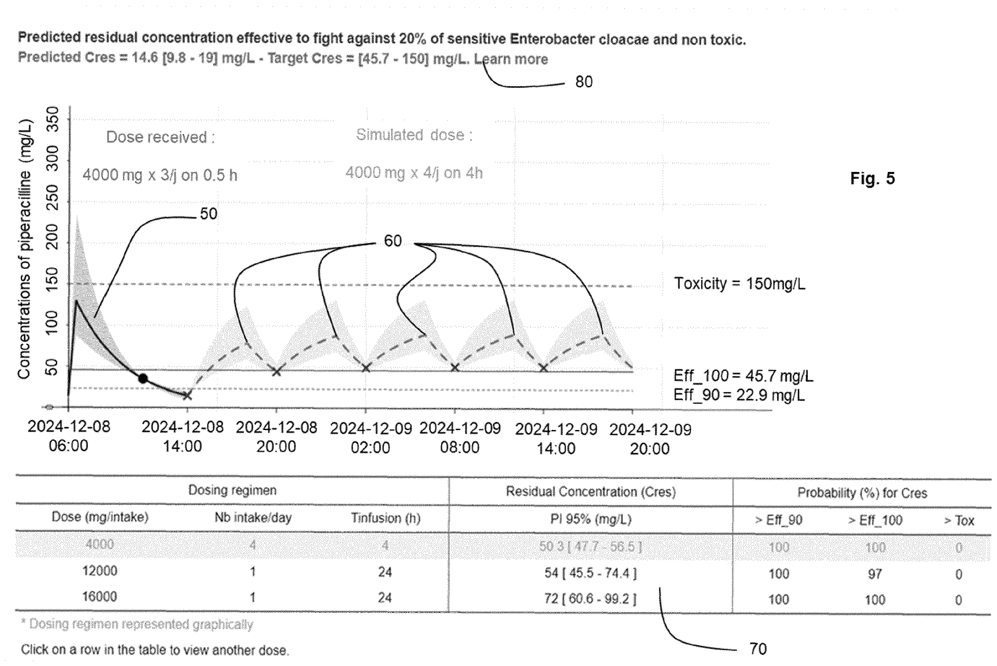

These results are displayed to the physician, by giving him
a. an interval of effective and non-toxic concentrations (with an explanation of the way to obtain this interval), as shown in **figure 5****,** second line above the table.
b. evaluation of this concentration (i.e. the dose used) from an efficacy point of view (effective, effective to fight against n% of strains of a bacteria, non-effective) and/or in a toxicity point of view (non-toxic concentration, toxic concentration, high concentration i.e. higher than the reported concentrations in the literature) for a posteriori adaptation, as shown in **figure 5****,** first line, above the table. More information about the determination of the targets are available in the "Learn more" link (80)

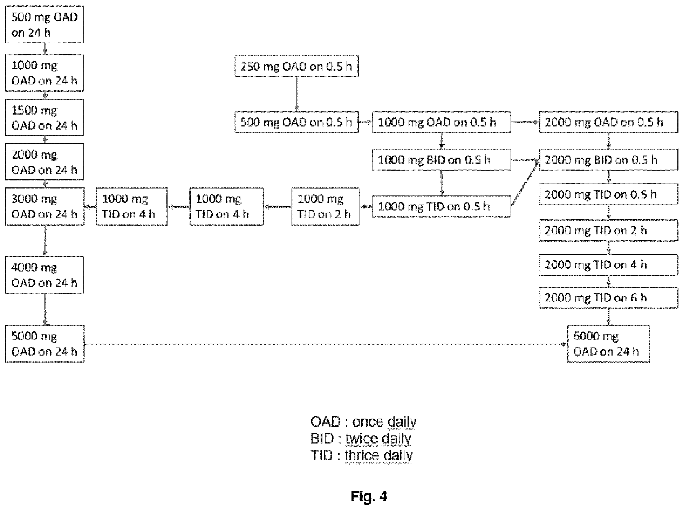

On a next step 500, for a selected drug, the method 10 according to the invention simulate different dosing scheme, ranging from the minimum daily dose (reported in the SPC or observed in clinical practice) to the maximum recommended dose in the SPC. For each drug, an algorithm, containing the most commonly used dosage indicates how to increase or decrease concentrations from an administration scheme to another, by varying dose, interval of use and duration of infusion (for intravenous administrations). For an a priori adaptation, simulation of a loading dose (dose, infusion duration if iv administration) is adding before maintenance dose, for continuous infusion. In renal impairment patient when recommended, a loading dose is adding to the maintenance dose, for the simulation. **Figure 4** illustrates an example of dose algorithm for cefepime in adult for an a posteriori adaptation according to the invention. This example is not limitative and could change with the evolution of the solution.

The simulations start on the date of sampling, except if the physician has completed data related to "Changes in doses and covariates since the last sampling", it will then begin on the date of the last re-administration entered and the modified dose and covariates will be taking into account in the simulations.

For an "a posteriori" adaptation :
- In the case of a sub-dosing, doses are increased as long as the maximum daily dose has not been reached and PPT or 97.5e percentile of PPT < toxicity target
- In the case of overdose, doses are decreased as long as the minimum daily dose has not been reached and as long as the 2.5e percentile of PPE > efficacy target
- For a normodosing or underdosing with toxicity risk, the doses are increased as for a sub-dosing and decrease as for an overdose by stopping according to the same rules described above.

In the "a priori" adaptation, the prediction intervals are much wider, the method 10 according to the invention is not limited to have 2.5% of patients below the efficacy target or above the toxicity target but to allow this value to go up to 20% (Other limits can be used according to situations). This reduces the dose as long as the patient's efficacy parameter under the efficacy target is < 20%. The dose is increased as long as the probability that the patient's toxicity parameter above the toxicity target is < 20%. Note that the target percentage of 20% is only given as an example and may be fixed to another value. These percentages can be modified.

For each dosing regimen or for the most optimal ones, are displayed in a table the PPE and the PPT if different and the probability for the PPE to be above the target efficacy and the probability for the PPT to be above the toxic target or the high dose target. The probability for the PPE to be above a lower efficacy target, effective above n % of the strains, could be determined if MIC distributions exist, as shown in **Figure 5****,** where n was fixed to 90.

The purpose of these simulations drawn by the method 10 according to the invention is to propose a set of dosage regimens for the physician so that he can make his choice in knowledge of the percentage of efficacy and toxicity or estimated high concentrations.

The results are displayed to the physician. For example, the method 10 according to the invention displays :
a. a time-dependent concentration graph obtained with
   i. a simulation dosing regimen for a priori adaptation (with or without a loading dose)
   ii. the given dose regimen (50) followed by simulated dosing regimen (60) for a posteriori adaptation, as shown in **Figure 5****.**
b. a table (70) listing different possible administration schemes reported in a line of said table with their PPE, PPT, probability for the PPE to be above the target efficacy and if exists above the efficacy n%, the PPT to be above the toxic target or the high dose target. Clicking to a line of the table allows to draw the time
   - concentration curve obtained with the dosing regimen proposed in this line. An example of table is shown in **Figure 5****.**

Preferably, the method 10 according to the invention is adapted to prompt the physician to input only the data which are specific to the patient to be treated, i.e. patient-related data concerning his characteristics, his administration scheme, the information on his infection, while general information concerning the drug, the pathogen suspected by type of infection, the susceptibility of the bacteria, the concentrations-effects relationships have been previously stored in the application. For that purpose, the method 10 according to the invention is adapted to update the general information from third-parties databases 6 by connecting to the application server 4 via its communication interface 3.4.

In an alternate embodiment wherein the method 10 according to the invention is executed directly on the application server 4, the method 10 according to the invention is adapted to send a data update request to the terminal 3, so that the user 1 can input patient-related data to be sent to the application server 4. This embodiment is particularly advantageous to strengthen patient's data privacy, in that his/her personal data are not stored directly on user terminal 3 but on a remote server that can be secured.

There exist population pharmacokinetic models of patients allowing to describe the concentration of a drug as a function of time for an average subject. A series of reference pharmacokinetic models may be stored on the application server 4 and/or retrieve from third-parties databases 6.

These models may be accessible online and retrieved by the terminal 3 upon request or may be initially stored in a memory 3.3 (ROM) of the terminal upon installation of the application corresponding to the method 10 according to the invention.

It is assumed that such a model (PopPK) is known for the drug molecule used to treat the patient. According to the present embodiment, this model is stored in the ROM 3.3 of the terminal 3.

The method 10 according to the invention is configured to adapt this pharmacokinetic model by taking into account relevant patient-related data including biological information (e.g. age, weight, *etc*) and clinical data, medical condition or infection status. For instance, if the patient is affected by renal failure, the pharmacokinetic model is adapted not only according to his/her weight but also according to his/her renal function.

For the "a posteriori" adaptation of the drug regimen, it is assumed that the drug concentration is measured in the patient's blood at an arbitrary time t. A significant advantage of the method 10 according to the invention is that it may be applied regardless of the time at which the drug concentration is measured in the patient's blood. It results therefrom that a drug dosing regimen may be determined with more flexibility, e.g. without having to observe specific times for taking measurements as conventionally required by existing dosing techniques.

According to an alternative embodiment, the method 10 according to the invention advantageously obtains a measured value of the drug concentration or information on the infection, for instance from the measuring device 7 and adapts automatically the patient's pharmacokinetic model according to this measured value.

This measurement may be performed by the measuring device 7 itself which is coupled to the terminal 3. In that case, the method 10 according to the invention is advantageously configured to receive automatically at least one measured value for the drug concentration or infection parameters.

More precisely, the method 10 according to the invention is configured to estimate the drug concentration over time by applying a known Bayesian model based on said measured value. This Bayesian model is preferably stored initially with the application on the terminal 3 or may be stored in a memory of the application server 4 and/or retrieved upon request from the application server 4.

Although the invention has been described hereinabove with reference to a specific embodiment, the present invention is not limited to this specific embodiment and modifications will be apparent to a person skilled in the art which as falling within the scope of the invention.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

## Claims

1. Method implemented by a computer for determining at least one optimal drug dosing regimen to treat a patient (2) efficiently against at least one drug-sensitive pathogenic agent responsible for infection, said method comprising the following steps:
a. obtaining patient-related data related to said patient (2), such as birth date, gender, weight, creatinemia, and co-treatments;
b. obtaining drug-related data, said drug-related data including parameters selected from :
i. the drug, the route of administration when several routes exist for said drug and the adaptation type as "a posteriori" or "a priori" ;
ii. the drug treatment regimen;
wherein the process comprises a next step of:
c. determine values for pharmacokinetic parameter for efficacy PPE and pharmacokinetic parameter for toxicity PPT from the patient related data, the drug related data and at least one population pharmacokinetic model for "a priori" adaptation and for "a posteriori" adaptation, when PPE and PPT are not obtained directly with the measured concentration(s);
d. obtaining infection related data comprising parameters selected from the documentation of infection, the infection localization, the type of infection, the pathogen if identified, the susceptibility of the pathogen to the antifungigram/antibiogram, and the MIC if determined;
wherein the process further comprises next steps of:
e. Determination of an efficacy target for the drug from at least the infection related data;
f. Determination of a target at risk of toxicity; and,
g. Determination of at least one optimal dosing regimen of the drug by comparison of pharmacokinetic parameter of efficacy PPE with the efficacy target and the pharmacokinetic parameter of toxicity PPT with the risk of toxicity target.

2. The method of claim 1, wherein, for "a posteriori" adaptation, drug-related data includes dates and times of drug administrations and sampling or the calculated or imposed interval between the administration and the sampling and measured drug concentration(s).

3. The method of claim 1 or 2, wherein the selection of the pathogen agent(s) is made according to:
• all pathogens considered to be drug sensitive with available susceptibility to the drug if the location is unknown and the infection is not documented;
• the main pathogens responsible for the infection if the location and the type of the infection are known but not documented, or
• the identified pathogen if the infection is documented.

4. The method according to claim 3, wherein the method comprises a subsequent step of determining the drug susceptibility SUS of the pathogen agent(s) from the selection step.

5. The method according to claim 4, wherein the susceptibility SUS determined is one of:
• MIC if determined; otherwise,
• Critical concentration "S≤" if the pathogen is sensitive to the drug on the antifungigraml/antibiogram; or,
• Critical concentration "R>" if the pathogen is sensitive to high dose; otherwise
• ECOFF if critical concentrations "S≤" and/or "R>" are unknown.

6. The method according to one of the claims 1 to 5, wherein the method comprises a step of determining the target of the drug among a group with at least one plasma target, a target at the level of the localization of the infection.

7. The method according to one of the claims 1 to 6, wherein the target is associated with the percentage of protein binding %lia of the drug if target is based on free fraction of the drug and/or the percentage of diffusion of the drug Diff to the infected site.

8. The method according to one of the claims 1 to 7, wherein the method comprises a step of determining a drug efficacy coefficient Ceff from published literature concentration/efficacy relationships or based on a selected target.

9. The method according to one of the claims 4 to 5 and the claims 6 or 7 and the claim 8, wherein the plasma efficacy target is determined based on the selected pathogen(s), associated susceptibility, determined target of the site and determined drug efficacy coefficient.

10. The method according to claim 9, wherein, for a plasma target, the plasma efficacy target is equal to SUS x Ceff, if the plasma target depends on a drug total form.

11. The method according to 9, wherein, for a plasma target, the plasma efficacy target is equal to SUS x Ceff/ (1-%lia), if the plasma target depends on a drug free form.

12. The method according to claim 9, wherein, for an infected site target, the plasma efficacy target is equal to SUS x Ceff / Diff.

13. The method according to claim 12, wherein the efficacy target is weighted by a percentage of protein binding of the drug at the localization of infection.

14. The method according to one of the claims 1 to 13, wherein an efficacy target for controlling n% of the pathogen-susceptible strains is determined, if a MIC distribution for the pathogen/anti-infective pair is available or is calculated.

15. The method according to one of the claims 1 to 14, wherein the toxicity target is defined from a demonstrated concentration relationship toxicity, if known, otherwise the toxicity target is replaced by a high concentration by comparison to the literature reported concentrations.

16. The method according to one of the claims 1 to 15, wherein the drug dosing regimen, in the case of "a posteriori" adaptation, is the administered regimen to the patient, and, in the case of "a priori" adaptation, is the intended regimen to be administered or the regimen extracted from the summary of product characteristics adapted to the patient's characteristics or the regimen recommended by known expert committees.

17. The method according to one the claims 1 to 16, wherein the method comprise a further step of displaying :
a. glomerular filtration rate as formulated in the population pharmacokinetics publication and/or time to steady state, from patient related data and/or
b. a fit graph showing the adequacy of the measured concentration in the patient and the expected concentrations obtained by simulation of the population pharmacokinetic model applied to patient related data and drug related data, in the case of "a posteriori" adaptation and/or
c. a time-dependent concentration graph in the case of an "a posteriori" adaptation produced with the dose already administered followed by the simulated doses, or in the case of "a priori adaptation" produced with the dosing regimen according to claim 16 or produced with simulated doses and/or,
d. a table listing different possible administration schemes, their corresponding graphic being displayed when clicking on the line of the table corresponding to a specific scheme.

18. The method according to one of the claims 1 to 17, wherein the method comprises an additional step of simulating, for the drug, different dosing scheme, ranging from a minimum daily dose to a maximum recommended dose, by increasing or decreasing doses from an administration scheme to another, by varying dose, interval of use and for intravenous administration duration of infusion.

19. Computer programme comprising instructions adapted to implement the steps of the method according to any one of claims 1 to 18 when the programme is executed on a computer (3).
